# EUROPÄISCHE PATENTSCHRIFT

(11) **EP 3 117 895 B1**
(45) Veröffentlichungstag und Bekanntmachung des Hinweises auf die Patenterteilung: **29.01.2020**
(21) Anmeldenummer: 16178904.5
(22) Anmeldetag: 11.07.2016
(51) Int. Cl.: B01F 11/00, B01F 15/02, B01F 13/00, B01F 13/06, A61B 17/88

(54) **VAKUUMMISCHSYSTEM UND VERFAHREN ZUM MISCHEN VON POLYMETHYLMETHACRYLAT- KNOCHENZEMENT**
VACUUM MIXING SYSTEM AND METHOD FOR THE MIXING OF POLYMETHYLMETHACRYLATE BONE CEMENT
SYSTÈME DE MÉLANGE SOUS VIDE ET PROCÉDÉ DE MÉLANGE DE CIMENT OSSEUX EN POLYMÉTHACRYLATE DE MÉTHYLE

(30) Priorität: 13.07.2015 DE 102015111320
(43) Veröffentlichungstag der Anmeldung: 18.01.2017
(73) Patentinhaber: Heraeus Medical GmbH, 61273 Wehrheim (DE)
(72) Erfinder: Kluge, Thomas Dr., 56179 Vallendar (DE); Vogt, Sebastian Dr., 99092 Erfurt (DE)
(74) Vertreter: Schultheiss & Sterzel Patentanwälte PartG mbB

(56) Entgegenhaltungen:
- EP-A1- 2 957 337
- EP-A2- 0 796 653
- EP-A2- 2 730 332
- DE-B3-102009 031 178
- DE-U1- 20 005 333
- US-A1- 2010 091 606

## Beschreibung

Die Erfindung betrifft ein Vakuummischsystem zum Mischen von Polymethylmethacrylat-Knochenzement (PMMA-Knochenzement) aus zwei Ausgangskomponenten, insbesondere zum Mischen eines medizinischen Knochenzements, und zur Lagerung der Ausgangskomponenten. Die Erfindung betrifft ferner ein Verfahren zum Mischen von Polymethylmethacrylat-Knochenzement.

Gegenstand der Erfindung ist somit ein Vakuummischsystem für die Lagerung, Vermischung und gegebenenfalls auch den Austrag von Polymethylmethacrylat-Knochenzement. Weiterhin betrifft die Erfindung ein Verfahren zum Transfer von Monomerflüssigkeit in das Vakuummischsystem und ein Verfahren zum Vermischen der Komponenten von Polymethylmethacrlyat-Knochenzement.

Polymethylmethacrylat-Knochenzemente (PMMA-Knochenzemente) gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden mit der EP 0 692 229 A1, der DE 10 2009 031 178 B3, der US 5,997,544 A, der US 6,709,149 B1, der DE 698 12 726 T2 und der US 5,588,745 A vorgeschlagen. Auch bei diesen Mischsystemen ist eine externe Vakuumquelle notwendig. Das Patent DE 10 2009 031 178 B3 offenbart dabei eine Vakuummischvorrichtung mit einem zweiteiligen Austragskolben, der auch für eine erfindungsgemäße Vakuummischvorrichtung einsetzbar ist. Die DE 200 05 333 U1 offenbart ein Mischsystem entsprechend dem Oberbegriff des Anspruch 1. Sie offenbart eine Vorrichtung, an die eine manuell bedienbare Vakuum-Pumpe zum Erzeugen eines Unterdrucks anschließbar ist. Aus der EP 2 730 332 A2 ist ein Vakuummischsystem mit einem zweiteiligen Austragskolben in Form eines Dichtungskolbens und eines Sterilisationskolbens bekannt.

Bei der Verwendung von Vakuummischsystemen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Die EP 0 796 653 A2 offenbart ein System zum Mischen von Knochenzement, bei dem eine manuell bedienbare Pumpe und ein Monomerflüssigkeitsbehälter oben an eine Kartusche enthaltend Zementpulver angeschlossen werden. Nachteilig ist daran, dass das System zunächst zusammengesetzt und aufgestellt werden muss und dadurch die Bedienung aufwendig ist. Zudem kann die Monomerflüssigkeit schon vor dem Evakuieren des Innenraums der Kartusche in die Kartusche eindringen und dort vorzeitig mit dem Zementpulver reagieren. Hierdurch kann ein ungleichmäßiger Knochenzement entstehen. Zudem muss die Monomerflüssigkeit durch das Zementpulver sickern, um eine vollständige Durchmischung zu erreichen oder der Inhalt muss intensiv gemischt werden. Ferner können die tief im Zementpulver liegenden Bereiche nicht leicht evakuiert werden, wodurch im gemischten Knochenzementteig unerwünschte Lufteinschlüsse entstehen können.

Ein interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für einen Mischstab oder ein Mischrohr nicht ohne weiteres dauerhaft vakuumdicht ist.

Die Aufgabe der Erfindung besteht also darin, die Nachteile des Stands der Technik zu überwinden. Insbesondere sollen die Nachteile der bekannten Vakuummischsysteme mit externer Vakuumquelle überwunden werden, ohne dass ein Unterdruck über lange Zeit gehalten werden muss. Die Erfindung hat insbesondere die Aufgabe ein Vakuummischsystem zu entwickeln, bei dem erst unmittelbar vor dem Vermischen der Zementkomponenten ein Unterdruck erzeugt wird. Die Vorrichtung soll maximal vereinfacht sein und es erlauben, zumindest einmalig einen Unterdruck in einer Zementkartusche gegenüber der umgebenden Atmosphäre zu erzeugen. Weiterhin kann es vorteilhaft sein, wenn das Vakuummischsystem in der Lage ist, einen Transfer von Monomerflüssigkeit aus einem Monomerbehälter in eine mit Zementpulver gefüllte Kartusche zu ermöglichen. Es soll dann ferner ein Verfahren bereitgestellt werden, das einen Monomertransfer und ein Vakuummischen in Full-Prepacked-Mischsystemen ermöglicht. Weiterhin soll das zu entwickelnde Vakuummischsystem, hauptsächlich aus kostengünstigem Kunststoff gefertigt werden können.

Die Erfindung hat ferner die Aufgabe, eine einfache geschlossene Vorrichtung zu entwickeln, bei der Polymethylmethacrylat-Knochenzementpulver und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Vorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit muss ausgeschlossen sein. Bei der zu entwickelnden Vorrichtung handelt es sich um ein Full-Prepacked Mischsystem. Die Vorrichtung soll so beschaffen sein, dass ein Transfer der Monomerflüssigkeit in das Polymethylmethacrylat-Knochenzementpulver durch Vakuum ohne die Verwendung von externen Vakuumpumpen erfolgt. Des Weiteren soll die Vorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Besonders bevorzugt soll die Vorrichtung möglichst weitgehend auch ohne interne Energiespeicher, wie beispielsweise Batterien oder mechanische Energiespeicher auskommen Die Vorrichtung soll ohne zusätzliche technische Ausrüstung autonom verwendungsfähig sein.

Des Weiteren soll eine kostengünstig zu fertigende und zuverlässig funktionierende Vorrichtung zum Mischen eines medizinischen Zements und gegebenenfalls zur Lagerung der Ausgangskomponenten sowie ein Verfahren zum Mischen des Knochenzements gefunden werden, bei dem eine möglichst einfache manuelle Bedienung zum Mischen der Ausgangskomponenten angewendet werden kann, möglichst ohne dass Lufteinschlüsse in dem Mischgut entstehen können.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in dem Vakuummischsystem getrennt gelagert werden können und durch Anwendung der Vorrichtung sicher zusammengeführt werden können.

Die Aufgaben der Erfindung werden gelöst durch ein Vakuummischsystem zum Mischen von Polymethylmethacrylat-Knochenzement aufweisend zumindest eine Kartusche mit einem evakuierbaren Innenraum zum Mischen des Knochenzements, eine Pumpe mit einem manuell bewegbaren Kolben zum Erzeugen eines Unterdrucks und eine Verbindungsleitung, die den Innenraum der zumindest einen Kartusche mit der Pumpe zum Erzeugen eines Unterdrucks verbindet, wobei die Pumpe ein von außen bedienbares Bedienelement aufweist, das mit dem Kolben derart verbunden ist, dass es zum manuellen Bewegen des Kolbens in der Pumpe geeignet ist, so dass ein Unterdruck erzeugbar ist, so dass mit dem Unterdruck der Pumpe durch die Verbindungsleitung Gas aus dem Innenraum der zumindest einen Kartusche evakuierbar ist, wobei in der Kartusche ein Zementpulver enthalten ist und das Vakuummischsystem einen von der Kartusche separaten Behälter aufweist, in dem eine Monomerflüssigkeit enthalten ist, wobei der Behälter über eine Flüssigkeitsleitung mit der Kartusche verbunden ist, wobei die Flüssigkeitsleitung an der Vorderseite der Kartusche in die Kartusche einmündet und die Verbindungsleitung an der gegenüberliegenden Rückseite der Kartusche in die Kartusche einmündet, wobei die Kartusche, die Pumpe, der separate Behälter und alle Leitungen mit einem gemeinsamen Fußteil fest und/oder lösbar verbindbar sind, wobei der Kolben in einem Hohlzylinder axial beweglich gelagert ist, wobei der Hohlzylinder auf einer ersten Seite durch einen Verschluss geschlossen ist oder bis auf eine Durchführung für eine mit dem Bedienelement und dem Kolben verbundene Stange durch einen Verschluss geschlossen ist und der Hohlzylinder auf einer zweiten Seite offen ist, wobei ein Pumpraum in dem Hohlzylinder zwischen dem Kolben und der ersten geschlossenen Seite gebildet ist und wobei nach einem vollständigen Hub des Kolbens innerhalb des Hohlzylinders der Kolben so verschoben ist, dass das Volumen des vom Hohlzylinder, dem Verschluss und dem Kolben gebildeten Pumpraums mindestens gleich dem Volumen des Innenraums der zu evakuierenden Kartusche ist.

Der Vorderseite der Kartusche ist bei bestimmungsgemäßer Aufstellung des Vakuummischsystems unten angeordnet und die Rückseite dementsprechend oben. Dadurch liegt das Zementpulver unten auf der Vorderseite der Kartusche innen auf und die Monomerflüssigkeit wird mit dem Unterdruck von unten durch das Zementpulver in Richtung der Verbindungsleitung nach oben gezogen. Hierdurch wird eine gute Durchmischung des Zementpulvers mit der Monomerflüssigkeit erreicht und es wird gleichzeitig sichergestellt, dass die Mischung des Zementpulvers mit der Monomerflüssigkeit mit reduziertem Druck durchgeführt wird, so dass die Gefahr für Lufteinschlüsse im Knochenzementteig reduziert ist.

Theoretisch können auch mehrere bewegbare Kolben vorgesehen sein, von denen wenigstens einer manuell bewegbar ist. Im Sinne der vorliegenden Erfindung ist dann der manuell bewegbare Kolben zumindest einer der mehreren bewegbaren Kolben.

Im Sinne der vorliegenden Erfindung kann ein manuell bewegbarer Kolben auch durch eine manuell bewegliche Seitenwand realisiert werden. Wichtig ist nur, dass der Kolben durch einen manuellen Antrieb das Volumen in der Pumpe derart vergrößert, dass in der Pumpe ein Unterdruck entsteht, mit dem gepumpt werden kann.

Bevorzugt kann vorgesehen sein, dass der Kolben manuell axial in einem Hohlzylinder in der Pumpe zu verschieben ist.

Der Begriff Unterdruck bezieht sich vorliegend immer auf einen relativ zur umgebenden Atmosphäre bezogenen Druck, der kleiner ist als der umgebende Atmosphärendruck.

Bevorzugt kann vorgesehen sein, dass die Pumpe in das Vakuummischsystem integriert ist.

Der Polymethylmethacrylat-Knochenzement wird bevorzugt aus zumindest zwei Komponenten gemischt beziehungsweise ist aus zumindest zwei Komponenten herstellbar. Dabei ist eine Komponente die flüssige Monomerflüssigkeit und eine andere Komponente das Zementpulver.

Bevorzugt kann vorgesehen sein, dass der Druck in dem Innenraum der zumindest einen Kartusche durch den Pumpvorgang um wenigstens 50% reduzierbar ist, bevorzugt um wenigstens 90% reduzierbar ist.

Die Ausgangskomponenten für das Mischgut, insbesondere für den PMMA-Knochenzement, sind erfindungsgemäß bereits in den Kartuschen enthalten.

Die Vorrichtung ist erfindungsgemäß bevorzugt auch zum Lagern der Ausgangskomponenten geeignet, insbesondere dann, wenn die Behälter in die Vorrichtung eingesetzt sind oder die Behälter ein fester Teil der Vorrichtung sind.

Besonders bevorzugt ist das Mischgut ein Knochenzement, insbesondere ein PMMA-Knochenzement.

Mit einer Weiterbildung der Erfindung kann auch vorgesehen sein, dass mit dem Unterdruck durch die Verbindungsleitung Gas aus dem Innenraum der zumindest einen Kartusche evakuierbar ist und Gas aus einer Leitung zwischen dem Innenraum der Kartusche und dem Behälter evakuierbar ist und mit dem Unterdruck eine mit dem Zementpulver als erster Komponente des PMMA-Knochenzements in der Kartusche zu mischenden Monomerflüssigkeit aus dem Behälter in den Innenraum der Kartusche zu ziehen ist.

Bei erfindungsgemäßen Vakuummischsystemen kann vorgesehen sein, dass die Pumpe im Inneren einen gasdichten Pumpraum aufweist und in der Pumpe der bewegliche Kolben angeordnet ist, der den Pumpraum begrenzt, wobei der Kolben manuell in einer Richtung, bevorzugt unidirektional, antreibbar ist, so dass durch die Bewegung des Kolbens der Pumpraum vergrößert wird und mit dem dadurch im Pumpraum entstehenden Unterdruck der Innenraum der zumindest einen Kartusche durch die Verbindungsleitung evakuierbar ist.

Aufgrund der speziellen Anforderungen, wie die des geringen Volumens des Innenraums der Kartusche, sind aufwendigere Pumpensysteme nicht notwendig.

Dabei kann vorgesehen sein, dass die Volumenvergrößerung des Pumpraums mindestens so groß ist wie das freie Volumen des Innenraums der Kartusche, bevorzugt die Volumenvergrößerung des Pumpraums mindestens so groß ist wie die Summe des Volumens des Innenraums der Kartusche und des Volumens der Verbindungsleitung und des Volumens einer Leitung zwischen dem Innenraum und dem Behälter und des Volumens einer mit dem Zementpulver in der Kartusche zu mischenden Monomerflüssigkeit in dem Behälter.

Hierdurch wird sichergestellt, dass die Pumpe den Innenraum der Kartusche evakuieren kann. Dabei ist das Volumen des Pumpraums vor dem Pumpvorgang idealerweise möglichst klein. Es kann also bevorzugt vorgesehen sein, dass das Volumen des Pumpraums nach dem Pumpvorgang zumindest 10 mal größer ist als das Volumen des Pumpraums vor dem Pumpvorgang, besonders bevorzugt zumindest 20 mal größer ist als das Volumen des Pumpraums vor dem Pumpvorgang. Bevorzugt ist, dass der Kolben nicht an dem Innenraum der Pumpe, insbesondere an der Deckfläche eines Hohlzylinders mit dem Anschluss für die Verbindungsleitung, flächenbündig anliegt, damit sich der Kolben nicht an dieser Fläche festsaugt und nicht zum Starten der Bewegung nur schwer bewegt werden kann.

Das Zementpulver ist die erste (pulverförmige) Komponente des PMMA-Knochenzements und die Monomerflüssigkeit ist die zweite Komponente des PMMA-Knochenzements.

Ferner kann vorgesehen sein, dass das Vakuummischsystem eine Mischvorrichtung zum Mischen des Inhalts der zumindest einen Kartusche aufweist, wobei bevorzugt die Mischvorrichtung im Innenraum der Kartusche angeordnet ist und manuell oder durch einen Motor antreibbar ist.

Bevorzugt weist die Kartusche eine druckdichte Durchführung auf, durch die ein Stab oder ein Mischrohr durchgeführt ist, mit dem die Mischvorrichtung von außerhalb der Kartusche zu bedienen ist. Dazu ist der Stab oder das Mischrohr bevorzugt drehbar und in Längsrichtung verschiebbar in der Durchführung gelagert. Mit der Mischvorrichtung kann der Inhalt der Kartusche gut durchmischt werden.

Bevorzugte Ausführungsformen können sich dadurch auszeichnen, dass das Vakuummischsystem weniger als 30 kg Gesamtgewicht hat, besonders bevorzugt weniger als 10 kg Gesamtgewicht hat.

Diese geringen Gewichte sind mit dem erfindungsgemäßen Aufbau der Mischvorrichtung mit manuellem bedienbarem Bedienelement und der Pumpe möglich. Das geringe Gewicht hat den Vorteil, dass die Mischvorrichtung mitgenommen werden kann und ohne Anschluss an Versorgungsleitungen und ohne große Vorbereitungen einsetzbar ist.

Des Weiteren ist vorgesehen, dass der Kolben in einem Hohlzylinder axial beweglich gelagert ist, wobei der Hohlzylinder auf einer ersten Seite geschlossen ist oder bis auf eine Durchführung für eine mit dem Bedienelement und dem Kolben verbundene Stange durch einen Verschluss geschlossen ist und der Hohlzylinder auf einer zweiten Seite offen ist, wobei ein Pumpraum in dem Hohlzylinder zwischen dem Kolben und der ersten geschlossenen Seite gebildet ist.

Hierdurch wird durch die zylindrische Geometrie eine besonders einfach und kostengünstig zu fertigende Pumpe vorgeschlagen, die leicht zu bedienen ist und die besonders unanfällig für Fehlfunktionen ist.

Auch kann vorgesehen sein, dass der Kolben über eine Stange mit dem Bedienelement verbunden ist und vorzugsweise der Kolben durch Drücken des Bedienelements in der Pumpe zu bewegen ist.

Hierdurch wird ein besonders einfaches Vakuummischsystem bereitgestellt, bei dem keine große Gefahr für mögliche Störungen besteht. Die direkte Verbindung des Bedienelements mit dem Kolben über die Stange kann mit einem einteiligen Spritzgussteil aus Kunststoff realisiert werden. Alternativ kann auch eine Übersetzung beziehungsweise ein Getriebe vorgesehen sein, mit dem die Kraft, die auf das Bedienelement ausgeübt wird, zum Kolben übersetzt wird, um eine kräftiger Bewegung des Kolbens zu ermöglichen.

Mit einer Weiterbildung kann vorgesehen sein, dass in dem Innenraum der Kartusche ein beweglicher Austragskolben zum Austragen des gemischten Knochenzements aus der Kartusche angeordnet ist, wobei der Austragskolben vorzugsweise lösbar arretiert oder arretierbar ist, um eine Bewegung des Austragskolbens unter Einwirkung des Unterdrucks zu verhindern.

Mit dem Austragskolben wird die Bedienung des Vakuummischsystems vereinfacht.

Dabei kann vorgesehen sein, dass der Austragskolben einen Durchgang mit einer gasdurchlässige Porenscheibe aufweist, die für das Zementpulver undurchlässig ist, wobei der Durchgang mit der Porenscheibe den Innenraum der Kartusche mit der Umgebung gasdurchlässig verbindet, wobei der Durchgang gasdicht verschließbar ist, vorzugsweise mit einem Dichtungskolben des Austragskolbens gasdicht verschließbar ist.

Mit der Porenscheibe kann sichergestellt werden, dass der Innenraum der Kartusche mit dem Knochenzementpulver darin mit Hilfe eines Gases wie Ethylenoxid sterilisiert werden kann, ohne dass die Gefahr besteht, dass das Knochenzementpulver aus dem Innenraum der Kartusche nach außen in die Umgebung gelangt.

Ferner kann auch vorgesehen sein, dass der Austragskolben eine Durchführung enthält, in der sich eine manuell von außen zu betätigender Mischstab befindet, wobei im Innenraum der Kartusche eine Mischvorrichtung, insbesondere umfassend mehrere Mischflügel, an dem Mischstab befestigt ist, mit der der Inhalt des Innenraums der Kartusche durchmischbar ist.

Hiermit kann auf einfache Weise eine manuelle Durchmischung des Inhalts der Kartusche durchgeführt werden.

Bevorzugt kann vorgesehen sein, dass der Austragskolben an der Rückseite der Kartusche angeordnet ist.

Mit einer Weiterentwicklung wird vorgeschlagen, dass die Kartusche eine mit dem Zementpulver gefüllte Zementkartusche ist, wobei vorzugsweise der Behälter durch ein zu öffnendes Trennelement flüssigkeitsundurchlässig mit dem Innenraum der Zementkartusche verbunden ist und/oder der Innenraum der Zementkartusche mit der Pumpe gasdurchlässig verbunden ist oder verbindbar ist.

Hiermit wird erreicht, dass die Monomerflüssigkeit aus dem separaten Behälter mit der gleichen Pumpbewegung beziehungsweise dem gleichen Pumpvorgang in den Innenraum der Kartusche überführt werden kann, mit dem der Innenraum der Kartusche evakuiert wird. Das Vakuum beziehungsweise der Unterdruck, der mit der Pumpe manuell erzeugt wird, wird dabei gleichzeitig zum Einsaugen der Monomerflüssigkeit in die Kartusche genutzt.

Es ist vorgesehen, dass die Kartusche, die Pumpe, der separate Behälter und alle Verbindungsleitungen mit einem gemeinsamen Fußteil fest und/oder lösbar verbunden sind, wobei vorzugsweise die Pumpe, der separate Behälter und alle Verbindungsleitungen fest mit dem Fußteil verbunden sind und die Kartusche lösbar mit dem Fußteil verbunden ist.

Ein derartiges Vakuummischsystem kann einfach aufgestellt werden und ist leicht bedienbar. Die Anwendung des Vakuummischsystems wird dadurch vereinfacht. Am Ort der Anwendung muss dann lediglich eine ebene Unterlage zum Aufstellen des Vakuummischsystems vorhanden sein, was in den meisten OP-Bereichen kein Problem darstellt.

Ferner kann vorgesehen sein, dass die Flüssigkeitsleitung, die zwischen dem separaten Behälter und dem Innenraum der Kartusche vorgesehen ist, eine nach oben weisende Schlaufe aufweist, wobei der höchste Punkt der Schlaufe oberhalb einer Einmündung unter einem Monomer-Behälter liegt, der in dem separaten Behälter angeordnet ist.

Bei korrekter Aufstellung des Vakuummischsystems kann so sichergestellt werden, dass die Monomerflüssigkeit nicht ohne Einwirkung des Unterdrucks ungewollt in die Kartusche fließen kann und dort ungewollt aushärtet. Damit kann verhindert werden, dass die Monomerflüssigkeit beim Einfüllen in den separaten Behälter beziehungsweise beim Öffnen des Monomer-Behälters (der Monomer-Glasampulle) in dem Behälter bereits durch die Flüssigkeitsleitung in den Innenraum der Kartusche gelangt. Diese umgekehrt U-förmige Schlaufe der Flüssigkeitsleitung erreicht, dass vor Bewegung des Pumpkolbens die Monomerflüssigkeit im Behälter bis zur Höhe des Scheitelpunkts in der Flüssigkeitsleitung verbleibt, wodurch ein vorzeitiger Eintritt der Monomerflüssigkeit zum Zementpulver verhindert wird. Insbesondere bei hochviskosen Zementen kann ein vorzeitiger Kontakt von schon geringen Volumina der Monomerflüssigkeit mit dem Zementpulver zur Verklebung der Flüssigkeitsleitung oder eines als Düse ausgebildeten Leitungsmittels, wie in US 8 662 736 B2 beschrieben, führen. Die Flüssigkeitsleitung kann transparent oder transluzent sein, damit der Anwender den Monomertransfer visuell kontrollieren kann. Hierzu kann insbesondere ein Sichtfenster in der Vorrichtung vorgesehen sein, durch das die Schlaufe mit dem höchsten Scheitelpunkt zu erkennen ist.

Ein erfindungsgemäßes Vakuummischsystem kann sich dadurch auszeichnen, dass die Pumpe aufgebaut ist mit einem Hohlzylinder, wobei der Hohlzylinder mit dem Innenraum der Kartusche verbunden oder verbindbar ist, einem gasdichten Verschluss an einem Hohlzylinderende, einem Kolben, der im Hohlzylinder gasdicht, axial beweglich angeordnet ist, mindestens einem manuell bedienbaren Bedienelement, mit dem der Kolben in der Pumpe manuell bewegbar ist, wobei bei einem Bewegen des Kolbens mit dem mindestens einem manuell bedienbaren Bedienelement der Kolben axial entgegengesetzt zum Verschluss bewegbar ist und dadurch Gas aus dem Innenraum der Kartusche evakuiert.

Dieser Aufbau ist besonders einfach und die Teile hierfür können aus Kunststoff durch Spritzgießen gefertigt werden.

Des Weiteren kann vorgesehen sein, dass nach einem vollständigen Hub des Kolbens innerhalb des Hohlzylinders der Kolben so verschoben ist, dass das Volumen des vom Hohlzylinder, dem Verschluss und dem Kolben gebildeten Pumpraums mindestens gleich dem Volumen des Innenraums der zu evakuierenden Kartusche ist.

Durch die Abstimmung der Volumina wird erreicht, dass die Pumpe für den genannten Zweck ausreichend dimensioniert ist.

Die der vorliegenden Erfindung zugrundeliegenden Aufgaben werden auch gelöst durch ein Verfahren zur Vermischung von Polymethylmethacrylat-Knochenzement in einem Innenraum einer Kartusche eines erfindungsgemäßen Vakuummischsystems, bei dem eine Bewegung eines manuell angetriebenen Kolbens einer Pumpe des Vakuummischsystems zum Erzeugen eines Unterdrucks in der Pumpe verwendet wird, wobei mit der derart angetriebenen Pumpe der Innenraum der Kartusche von oben evakuiert wird, wobei ein Zementpulver in dem Innenraum der Kartusche enthalten ist und ein Gas aus dem Innenraum der Kartusche mit der Pumpe evakuiert wird, wobei mit dem Unterdruck eine Monomerflüssigkeit von unten in den Innenraum der Kartusche eingeleitet wird und anschließend in dem Innenraum der Kartusche die Monomerflüssigkeit mit dem Zementpulver in dem evakuierten Innenraum der Kartusche zu einem Knochenzement gemischt wird.

Bevorzugt wird zum Erzeugen des Unterdrucks mit der Pumpe nur ein einziger Hub des Kolbens der Pumpe durchgeführt. Nach dem Mischen des Knochenzements kann der gemischte Knochenzementteig mit einem Austragskolben aus der Kartusche ausgepresste werden. Bevorzugt wird die Kartusche zuvor von der Pumpe getrennt.

Bei erfindungsgemäßen Verfahren kann vorgesehen sein, dass das Volumen eines Pumpraums der Pumpe durch die manuelle Bewegung des Kolbens vergrößert wird und durch den dadurch entstehenden Unterdruck der Innenraum der Kartusche evakuiert wird.

Des Weiteren kann vorgesehen sein, dass die Monomerflüssigkeit mit dem Unterdruck der Pumpe in den Innenraum der Kartusche gesaugt wird, wobei vorzugsweise die Monomerflüssigkeit von unten durch das Zementpulver gezogen wird.

Ferner kann vorgesehen sein, dass der Kolben der Pumpe manuell bewegt wird, wodurch ein Unterdruck gegenüber der umgebenden Atmosphäre in der Pumpe erzeugt wird, dabei durch eine Verbindungsleitung Gas von oben aus dem Innenraum der Kartusche in den Hohlzylinder gesaugt wird und die Monomerflüssigkeit von unten in die Kartusche und in das Zementpulver gesaugt wird, anschließend manuell oder motorgetrieben mit einer Mischvorrichtung das Zementpulver mit der Monomerflüssigkeit vermischt wird, anschließend die Kartusche mit dem gemischten Zementteig entnommen wird und durch axiale Bewegung eines Austragskolbens der Zementteig aus der Kartusche gepresst wird.

Es kann auch vorgesehen sein, dass die Monomerflüssigkeit in einem von der Kartusche separaten Behälter angeordnet ist, wobei die Monomerflüssigkeit in einer Glasampulle enthalten ist, die Glasampulle geöffnet wird, bevor der Kolben manuell angetrieben wird, so dass eine flüssigkeitsdurchlässige Verbindung zwischen dem Innenraum der Kartusche und dem Behälter gebildet wird, danach der Kolben axial im Hohlzylinder bewegt wird, wodurch ein Unterdruck gegenüber der umgebenden Atmosphäre erzeugt wird, dabei durch die Verbindungsleitung Gas aus dem Innenraum der Kartusche in den Hohlzylinder gesaugt wird und durch den in dem Innenraum der Kartusche gebildeten Unterdruck Monomerflüssigkeit in die Kartusche gesaugt wird.

Der Erfindung liegt die überraschende Erkenntnis zugrunde, dass es mit Hilfe einer manuell anzutreibenden Pumpe gelingt, ein von inneren und äußeren Energiequellen und anderen Versorgungsleitungen unabhängiges Vakuummischsystem bereitzustellen. Das erfindungsgemäße Vakuummischsystem kann kompakt, leicht und platzsparend aufgebaut werden. Die Pumpe kann mit einfachsten Mitteln aufgebaut werden, so dass das gesamte Vakuummischsystem als Einmalsystem verwendet werden kann. Die Pumpe wird ferner dazu eingesetzt, um eine Monomerflüssigkeit in das Zementpulver zu überführen. Die beiden Komponenten des PMMA-Knochenzements können dann im Vakuum beziehungsweise in dem Unterdruck gemischt werden. Durch die erfindungsgemäße Anordnung der Verbindungsleitung zur Pumpe und der Flüssigkeitsleitung zum Behälter kann sichergestellt werden, dass der Knochenzementteig im Unterdruck blasenfrei und homogen gemischt werden kann.

Das erfindungsgemäße Vakuummischsystem hat ferner den Vorteil, dass die aus der Kartusche evakuierten Gase nicht an die Umgebung abgegeben werden, da diese Gase dann nicht gefiltert werden müssen, um unerwünschte Bestandteile (wie beispielsweise Methylmethacrylat-Dämpfe) zu entfernen. Stattdessen verbleiben die Gase einfach in der Pumpe beziehungsweise in dem Pumpraum.

In Zementiersystemen nach der vorliegenden Erfindung ist eine Vorrichtung zur Erzeugung eines Vakuums beziehungsweise zur Erzeugung eines Unterdrucks enthalten, die zur temporären Erzeugung eines Unterdrucks vor und während der Vermischung einer pulverförmigen Komponente mit einer flüssigen Monomerkomponente des Polymethylmethacrylat-Knochenzements geeignet ist.

Die der Erfindung zugrundeliegende Idee basiert auf der Erkenntnis, dass nur eine relativ geringe Energiemenge und damit ein geringer manueller Kraftaufwand notwendig ist, um das Vakuum beziehungsweise den Unterdruck in einer Kartusche zu erzeugen, der notwendig ist, um die Ausgangskomponenten eines Knochenzements unter dem Unterdruck oder dem Vakuum zu mischen. Die Energiemenge, um die Monomerflüssigkeit in das Zementpulver zu überführen, ist ebenfalls gering. Diese geringe Energiemenge kann ohne weiteres durch Eindrücken eines Kolbens in der Pumpe aufgebracht werden. Hierdurch ist das Vakuummischsystem einfach in der Handhabung und leicht zu bedienen sowie unabhängig von inneren und äußeren Energiespeichern.

Die Idee der Erfindung beruht auch darauf, dass durch manuelle Betätigung eines Bedienelements mit einem damit verbundenen Kolben in einem Hohlzylinder der Pumpe ein Unterdruck in dem Hohlzylinder der Pumpe erzeugt wird, wobei sich der Unterdruck über ein Leitungsmittel in die Kartusche ausbreitet, und die Monomerflüssigkeit aus einem geöffneten Monomerflüssigkeitsbehälter in die Kartusche gesaugt wird, in dem sich Zementpulver befindet. Danach erfolgt eine manuelle Vermischung der Zementkomponenten mit Hilfe eines Mischstabs und einer daran angebrachten Rührvorrichtung.

Der erfindungsgemäße manuell zu betätigende Monomertransfer durch Unterdruck kann kostengünstig mit einfachen, durch Kunststoffspritzgießen herzustellenden Kunststoffteilen realisiert werden. Der besondere Vorteil der erfindungsgemäßen Vorrichtung besteht darin, dass die Vorrichtung ohne äußere Hilfsmittel, wie durch Druckluft angetriebene Vakuumpumpen und Vakuumschläuche, und ohne Energiequellen, wie Druckluft oder Batterien, betrieben werden kann. Die erfindungsgemäße Vorrichtung ist autonom verwendbar und kann selbst unter einfachsten Operationsbedingungen verwendet werden. Mit der erfindungsgemäßen Vorrichtung wird ein geschlossenes Full-Prepacked-Zementiersystem für preissensitive Märkte zur Verfügung gestellt.

Im Folgenden werden weitere Ausführungsbeispiele der Erfindung anhand von sieben schematisch dargestellten Figuren erläutert, ohne jedoch dabei die Erfindung zu beschränken. Dabei zeigt:
- Figur 1:: eine schematische perspektivische Ansicht eines erfindungsgemäßen Vakuummischsystems vordem Pumpvorgang;
- Figur 2:: eine schematische Aufsicht auf das Vakuummischsystem nach Figur 1 mit drei Schnittebenen A-A, B-B und C-C;
- Figur 3:: eine schematische Querschnittansicht des Vakuummischsystems nach den Figuren 1 und 2 im Schnitt A-A nach Figur 2;
- Figur 4:: eine schematische Querschnittansicht eines Teils des Vakuummischsystems nach den Figuren 1 bis 3 im Schnitt B-B nach Figur 2;
- Figur 5:: eine schematische Querschnittansicht eines Teils des Vakuummischsystems nach den Figuren 1 bis 4 im Schnitt C-C nach Figur 2;
- Figur 6:: eine schematische Seitenansicht des Vakuummischsystems nach den Figuren 1 bis 5; und
- Figur 7:: eine schematische Querschnittansicht des Vakuummischsystems nach den Figuren 1 bis 6 im Schnitt A-A nach Figur 2 nach dem Pumpvorgang.

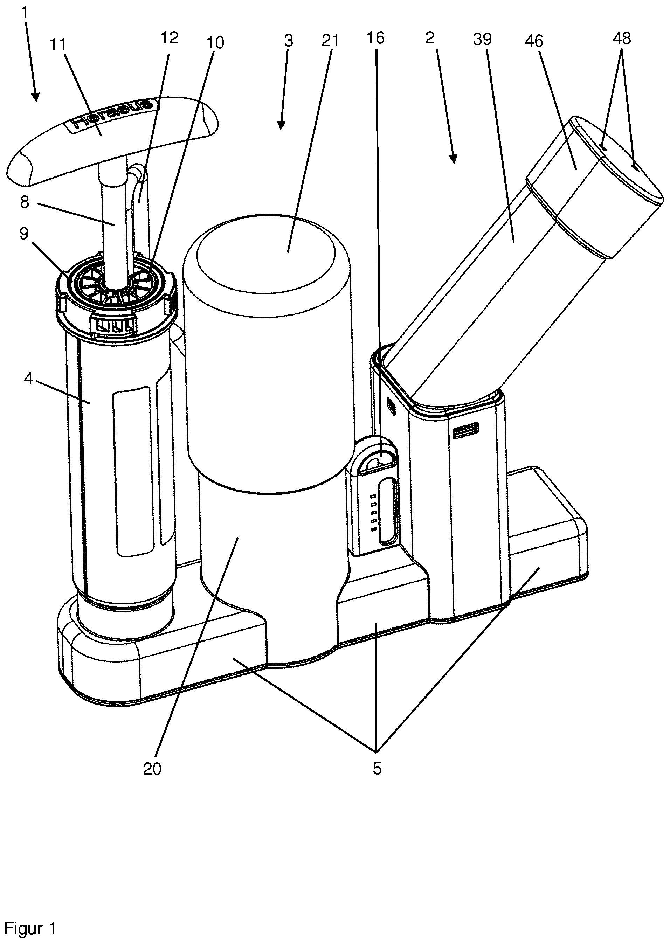

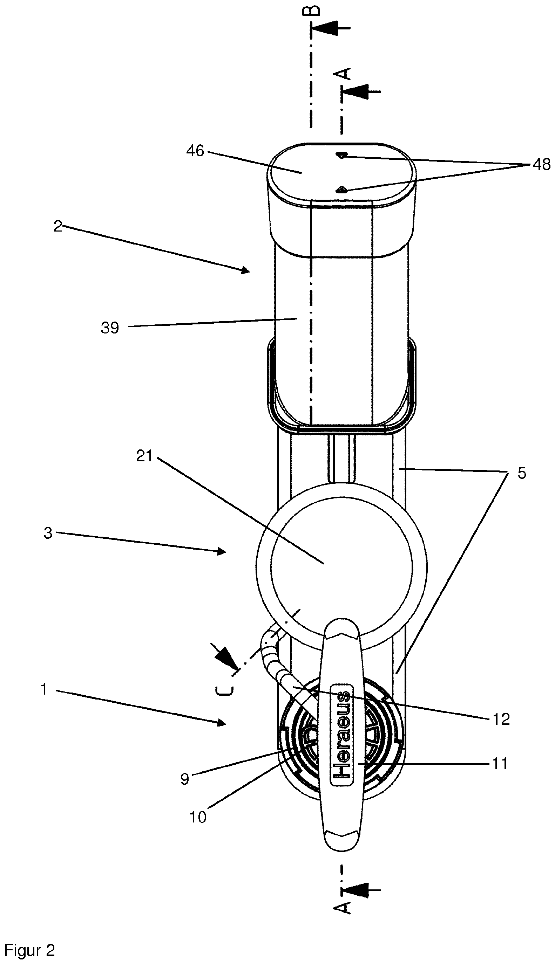

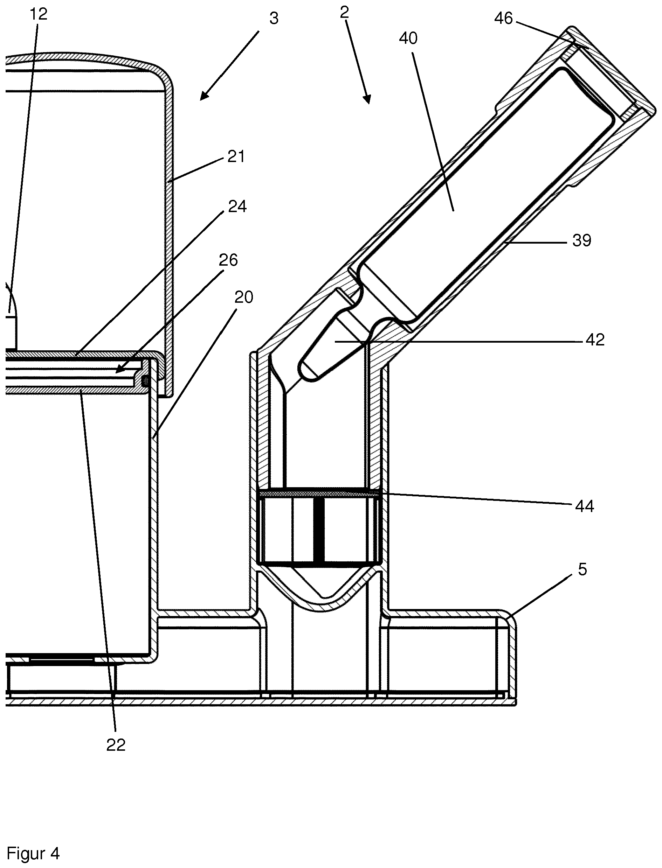

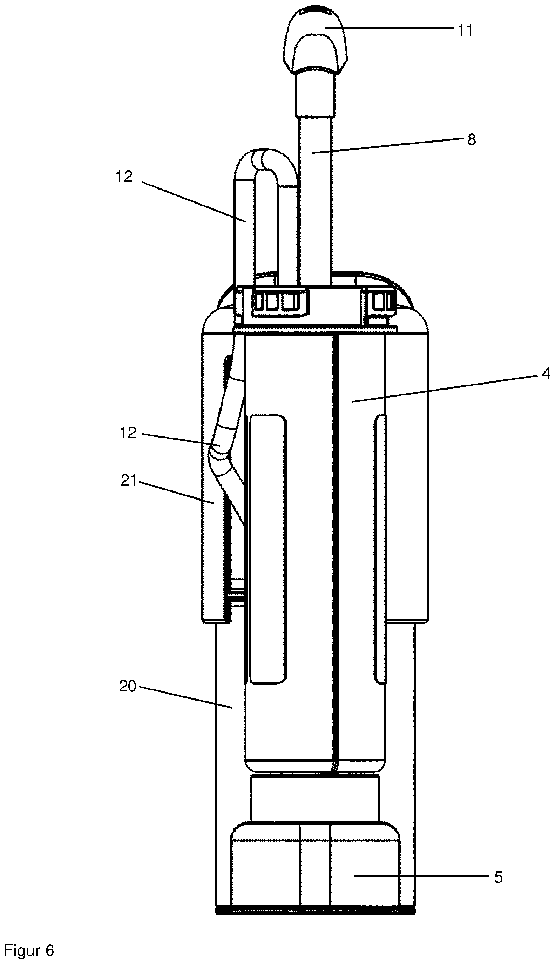

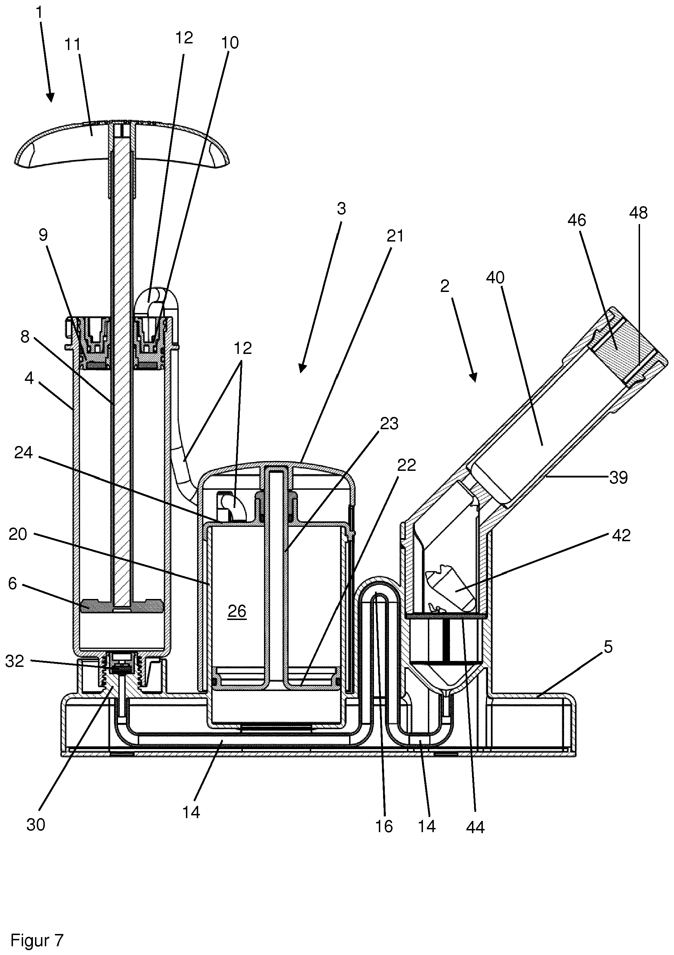

Die Figuren 1 bis 6 zeigen verschiedene Ansichten eines erfindungsgemäßen Vakuummischsystems vor dem Pumpvorgang. Figur 7 zeigt eine schematische Querschnittansicht des Vakuummischsystems nach dem Pumpvorgang. Das Vakuummischsystem besteht im Wesentlichen aus drei Teilen, einem Kartuschensystem 1, einem Flüssigkeitsbehälter 2 und einer manuell anzutreibenden Pumpe 3. Zentraler Bestandteil des Kartuschensystems 1 ist eine Kartusche 4, die mit einem Zementpulver (nicht gezeigt) für einen Knochenzement gefüllt ist. Das Kartuschensystem 1 ist mit dem Flüssigkeitsbehälter 2 und der Pumpe 3 über ein Fußteil 5 verbunden. Das Fußteil 5 bildet dabei unter anderem den Standfuß des kompakten Vakuummischsystems.

Die Kartusche 4 hat einen zylindrischen Innenraum mit kreisförmiger Grundfläche. Im Innenraum der Kartusche 4 ist ein Zementpulver enthalten. Zudem ist im Innenraum der Kartusche 4 eine Mischvorrichtung 6 mit zwei oder mehr Mischflügeln 6 angeordnet, die an einem Mischrohr 8 befestigt sind. Das Mischrohr 8 ist drehbar und in Längsrichtung verschiebbar durch einen Sterilisationskolben 9 hindurchgeführt, der sich an der oben angeordneten Rückseite der Kartusche 4 befindet. Die Durchführung ist hierzu druckdicht und gasdicht ausgeführt. Der Sterilisationskolben 9 weist eine Membran (nicht gezeigt) auf, die für ein sterilisierendes Gas durchlässig ist aber für das Zementpulver nicht durchlässig ist. Der Sterilisationskolben 9 wird nach dem Einfüllen des Zementpulvers in die Kartusche 4 eingesetzt und schließt den Innenraum der Kartusche 4 nach außen ab. Anschließend kann der Inhalt der Kartusche 4 durch die gasdurchlässige Membran mit Ethylendioxid sterilisiert werden.

Ein Dichtungskolben 10 kann in den Sterilisationskolben 9 gedrückt und mit diesem gas- und druckdicht verbunden werden. Die aneinander befestigten Kolben 9, 10 bilden dann zusammen einen Austragskolben 9, 10, mit dem der Inhalt der Kartusche 4 durch die bodenseitige Öffnung herausgepresst werden kann. Zunächst ist der Sterilisationskolben 9 aber an der Vorderseite mit der Öffnung gegenüberliegenden Seite (in Figuren 1, 3 und 5 bis 7 oben) arretiert, wobei die Arretierung lösbar ist.

An dem Mischrohr 8 ist außerhalb der Kartusche 4 ein Griffstück 11 befestigt, mit dem die Mischflügel 6 im Inneren der Kartusche 4, also im Innenraum der Kartusche 4 manuell drehbar und in Längsrichtung der Kartusche 4 verschiebbar sind.

In dem Dichtungskolben 10 ist eine Durchführung vorgesehen, die an eine Verbindungsleitung 12 in Form einer flexiblen Vakuumleitung 12 angeschlossen ist. Der Dichtungskolben 10 schließt ansonsten druckdicht mit der Kartusche 4. Die Vorderseite der Kartusche 4 (in den Figuren 1, 3 und 5 bis 7 unten) ist durch das Fußteil 5 über eine Flüssigkeitsleitung 14 druckdicht mit dem Flüssigkeitsbehälter 2 verbunden. In der Flüssigkeitsleitung 14 ist eine Schlaufe 16 als Siphon 16 vorgesehen, mit dem verhindert wird, dass ein in dem Flüssigkeitsbehälter 2 enthaltenes Monomer (nicht gezeigt) ungewollt bis in die Kartusche 4 vordringen kann.

Die Pumpe 3 weist einen stabilen Hohlzylinder 20 auf. Ein Bedienelement 21 ist an der Oberseite (in den Figuren 1, 3 und 5 bis 7 oben) der Pumpe 3 angeordnet, das manuell nach unten gedrückt werden kann. Der Hohlzylinder 20 ist über einen Kolben 22 druckdicht in zwei Teile getrennt. Hierzu weist der Kolben 22 eine umlaufende Dichtung auf, die mit der Innenwand des Hohlzylinders 20 abschließt. Der Kolben 22 ist einteilig mit einer Stange 23 ausgeführt, die durch eine druckdichte Durchführung in einem Verschluss 24 führt. Der Verschluss 24 schließt den Hohlzylinder 20 auf einer Seite druckdicht ab. Die Vakuumleitung 12 ist bis zu der Pumpe 3 geführt, so dass die Durchführung im Dichtungskolben 10 über die Vakuumleitung 12 druckdicht mit der Pumpe 3 genauer mit einem Pumpraum 26 der Pumpe 3 verbunden ist.

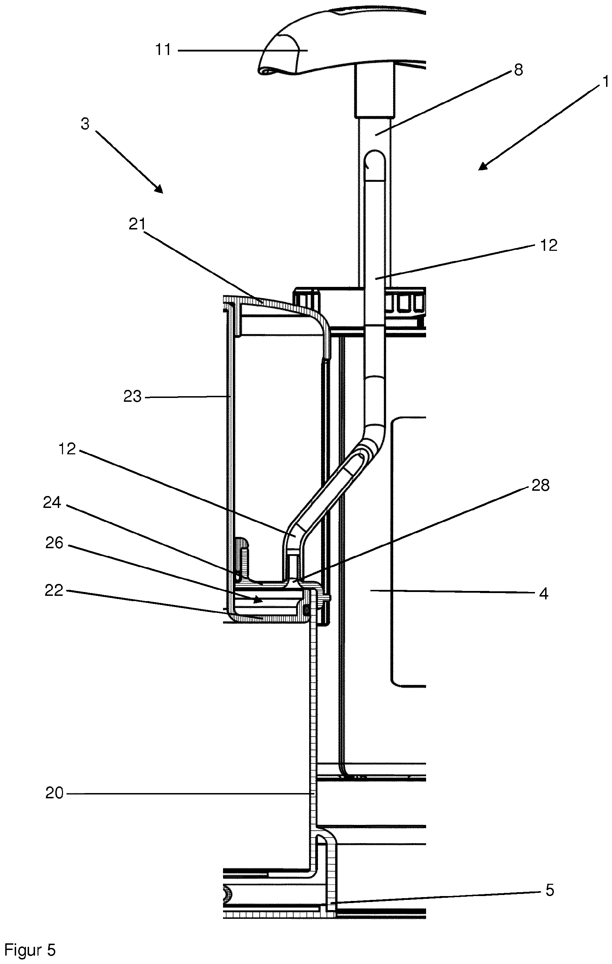

In dem Verschluss 24 am Hohlzylinder 20 ist eine Mündung 28 in die Vakuumleitung 12 beziehungsweise ein Anschluss 28 für die Vakuumleitung 12 vorgesehen (nur in Figur 5 zu sehen). Der Teil des Innenraums des Hohlzylinders 20 zwischen dem Verschluss 24 und dem Kolben 22 bildet den Pumpraum 26. Ein Unterdruck im Pumpraum 26 kann damit durch die Vakuumleitung 12 bis in den Innenraum der Kartusche 4 wirken, beziehungsweise ein Gas aus dem Innenraum der Kartusche 4 evakuiert werden, wenn der Dichtungskolben 10, wie in den Figuren gezeigt, mit dem Sterilisationskolben 9 verbunden ist und der Innenraum der Kartusche 4 dadurch nach außen bis auf die Öffnung zur Flüssigkeitsleitung 14 abgedichtet ist.

Die Kartusche 4 ist an dem Fußteil 5 senkrecht lösbar befestigt. Dazu ist an der Vorderseite der Kartusche 4 eine Öffnung mit einem Innengewinde vorgesehen, die auf einen Stutzen 30 mit einem Außengewinde am Fußteil 5 aufgeschraubt ist. Die Flüssigkeitsleitung 14 mündet in dem Stutzen 30 durch einen pulverundurchlässigen aber für die Monomerflüssigkeit durchlässigen Filter 32 in den Innenraum der Kartusche 4.

Der Flüssigkeitsbehälter 2 weist eine Aufnahme 39 für eine Glasampulle 40 auf. In dem Flüssigkeitsbehälter 2 ist eine Glasampulle 40 mit einem abbrechbaren Kopf 42 angeordnet. In der Glasampulle 40 ist die Monomerflüssigkeit enthalten. Die Aufnahme 39 für die Glasampulle 40 ist aus einem flexiblen Material wie beispielsweise Gummi gefertigt und kann manuell verbogen werden, um dem Kopf 42 der Glasampulle 40 manuell innerhalb des Flüssigkeitsbehälters 2 abbrechen zu können. Der Kopf 42 der Glasampulle 40 kann durch Verformen der Aufnahme 39 abgebrochen oder abgeschert werden. Hierzu weist die Aufnahme im Bereich des Halses zwischen dem Kopf 42 und dem Körper der Glasampulle 40 eine Verdickung auf. Nach dem Abbrechen des Kopfs 42 der Glasampulle 40 fließt die Monomerflüssigkeit aus der Glasampulle 40 aus. Möglicherweise entstehende Bruchstücke und Glassplitter sowie der abgebrochene Ampullenkopf 42 werden mit einem Sieb 44 oder Filter 44 zurückgehalten (siehe Figur 7). Zusätzlich kann am Eingang von dem Flüssigkeitsbehälter 2 in die Flüssigkeitsleitung 14 auch noch ein Ventilelement (nicht gezeigt) vorgesehen sein, das mit einem Drehhebel geöffnet werden kann.

Der Flüssigkeitsbehälter 2 wird mit einem Deckel 46 verschlossen, nachdem die Glasampulle 40 in den Flüssigkeitsbehälter 2 eingesetzt wurde. Damit die Monomerflüssigkeit ohne Probleme aus der Glasampulle 40 auslaufen beziehungsweise ablaufen kann, sind in dem Deckel 46 zwei Durchgänge 48 vorgesehen, durch die Luft von außerhalb in den Flüssigkeitsbehälter 2 nachströmen kann. Nach dem Aufbrechen der Glasampulle 40 ist die Monomerflüssigkeit in dem Flüssigkeitsbehälter 2 verfügbar und kann durch die Flüssigkeitsleitung 14 in den Innenraum der Kartusche 4 geleitet werden, in dem ein Unterdruck in den Innenraum der Kartusche 4 verwendet wird, um die Monomerflüssigkeit aus dem Flüssigkeitsbehälter 2 in den Innenraum der Kartusche 4 zu saugen. Dieser Unterdruck wird mit der Pumpe 3 erzeugt. Im Innenraum der Kartusche 4 kann die Monomerflüssigkeit dann mit dem Zementpulver mit der Mischvorrichtung 6 unter Vakuum beziehungsweise unter Unterdruck gemischt werden, um den Knochenzement beziehungsweise eine Knochenzementpaste zu erzeugen.

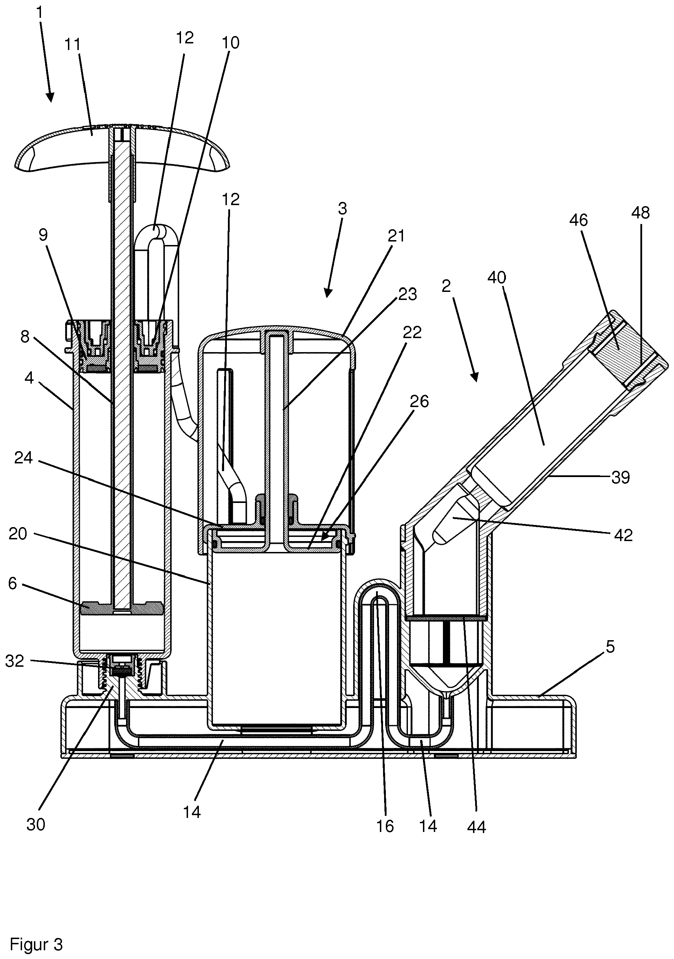

Das Vakuummischsystem zeichnet sich erfindungsgemäß durch die Anwendbarkeit des folgenden beispielhaften erfindungsgemäßen Verfahrens aus. Die Pumpe 3 wird verwendet, indem der Kolben 22 mit dem Bedienelement 21 manuell in den Hohlzylinder 20 gedrückt beziehungsweise gepresst wird. Dies wird gemacht, wenn die Kartusche 4 durch Einsetzen des Dichtungskolbens 10 einsatzbereit ist, wie in den Figuren gezeigt. Durch das Eindrücken wird der Kolben 22 wird in Richtung einer Öffnung im Boden des Hohlzylinders (in Figur 3 und 7 unten) bewegt. Durch diese Bewegung wird der Pumpraum 26 vergrößert. Dadurch wird der Druck im Pumpraum 26 verringert. Gas strömt aus der Vakuumleitung 12, dem Innenraum der Kartusche 4 und der Flüssigkeitsleitung 14 in den Pumpraum 26. Der Innenraum der Kartusche 4 wird so evakuiert.

Der Kolben 22 wird bis zum Ende des Hohlzylinders 20 (in den Figuren 3 und 7 unten) bewegt. Diese Anordnung ist in Figur 7 gezeigt. Die Volumenzunahme des Pumpraums 26 muss ausreichen, um das Gas aus der Vakuumleitung 12, dem Innenraum der Kartusche 4 und der Flüssigkeitsleitung 14 zu evakuieren und die Monomerflüssigkeit aus dem Flüssigkeitsbehälter 2 in den Innenraum der Kartusche 4 zu ziehen. Bevorzugt ist der expandierte Pumpraum 26, wie in Figur 3 gezeigt, zu diesem Zweck größer als die Volumina der Leitungen 12, 14, des Innenraums der Kartusche 4 und des Flüssigkeitsvolumens der Monomerflüssigkeit. Hierbei sei daran erinnert, dass die Figuren bezüglich der Größenverhältnisse des Pumpraums 26 und der anderen Volumina nur schematisch dargestellt sind.

Wenn die Ausgangskomponenten im Innenraum der Kartusche 4 mit den Mischflügeln 6 gemischt wurden, wird das Mischrohr 8 soweit es geht aus dem Innenraum der Kartusche 4 nach oben herausgezogen und kann dann an einer Sollbruchstelle abgebrochen werden. Der Dichtungskolben 10 wird gegen den Sterilisationskolben 9 gedreht und somit die Gasdurchführung durch den Dichtungskolben 10 verschlossen.

Die Vakuumleitung 12 wird vom Dichtungskolben 10 abgezogen. Die Kartusche 4 wird vom Fußteil 5 abgeschraubt und in das Innengewinde ein Austragsrohr (nicht gezeigt) eingeschraubt, durch das der gemischte Knochenzement appliziert werden kann. Der aus dem Sterilisationskolben 9 und dem Dichtungskolben 10 zusammengesetzte Förderkolben oder Austragskolben 9, 10 wird entrastet und kann mit einem Applikationsgerät (nicht gezeigt) ins Innere der Kartusche 4 getrieben werden. Dadurch wird der Inhalt der Kartusche 4, also der unter Unterdruck gemischte Knochenzement aus der gegenüberliegenden Öffnung und durch das aufgeschraubte Austragsrohr an der Vorderseite ausgepresst.

Die Bauteile des Vakuummischsystems können bis auf die Glasampulle 40, den Filter 32 und die Ausgangskomponenten des Knochenzements durch Spritzgießen aus einem Kunststoff gefertigt werden. Die Leitungen 12, 14 können aus einem anderen Kunststoff bestehen. Die Verbindungsleitung 12 muss flexibel sein, um den Dichtungskolben 10 beweglich auf dem Mischrohr 8 anordnen zu können und um eine Bewegung der Mündung 28 in dem Pumpraum zu ermöglichen. In dem Bedienelement 21 ist ein Längsschnitt vorgesehen, durch den die Verbindungsleitung 12 bei einer Bewegung des Bedienelements 21 und Kolbens 22 gleiten kann.

Die Leitungen 12, 14 sind in einem Gehäuse aus Kunststoff angeordnet, das das Fußteil 5 bildet, wobei das Fußteil 5 einen flachen Boden aufweist, damit das Vakuummischsystem auf einer flachen Unterlage aufgestellt werden kann.

Mit dem beschriebenen Vakuummischsystem können die beiden Ausgangskomponenten des Knochenzements gelagert und zu einem beliebigen späteren Zeitpunkt unter Vakuum gemischt werden. Dabei muss das Vakuummischsystem an keine externe Versorgung (Strom, Wasser oder Druckgas) angeschlossen werden. Es ist kein interner Energiespeicher, wie eine Batterie oder eine gespannte Feder, zum Antreiben des Vakuummischsystems notwendig. Die zum Erzeugen des Unterdrucks notwendige Energie wird ebenso manuell aufgebracht, wie die zum Öffnen der Glasampulle 40 notwendige Kraft.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartuschensystem
- 2: Flüssigkeitsbehälter
- 3: Pumpe
- 4: Kartusche
- 5: Fußteil
- 6: Mischflügel
- 8: Mischrohr
- 9: Sterilisationskolben
- 10: Dichtungskolben
- 11: Griffstück
- 12: Verbindungsleitung / Vakuumleitung
- 14: Flüssigkeitsleitung
- 16: Schlaufe / Siphon
- 20: Hohlzylinder
- 21: Bedienelement
- 22: Kolben
- 23: Stange / Rohr
- 24: Verschluss
- 26: Pumpraum
- 28: Mündung in die Verbindungsleitung
- 30: Stutzen mit Außengewinde
- 32: Pulverundurchlässiger und Flüssigkeitsdurchlässiger Filter
- 39: Aufnahme für Glasampulle
- 40: Glasampulle
- 42: Kopf der Glasampulle
- 44: Sieb / Filter
- 46: Deckel
- 48: Belüftungsdurchgang

## Patentansprüche

1. Vakuummischsystem zum Mischen von Polymethylmethacrylat-Knochenzement aufweisend
zumindest eine Kartusche (4) mit einem evakuierbaren Innenraum zum Mischen des Knochenzements,
eine Pumpe (3) mit einem manuell bewegbaren Kolben (22) zum Erzeugen eines Unterdrucks und
eine Verbindungsleitung (12), die den Innenraum der zumindest einen Kartusche (4) mit der Pumpe (3) zum Erzeugen eines Unterdrucks verbindet, wobei
die Pumpe (3) ein von außen bedienbares Bedienelement (21) aufweist, das mit dem Kolben (22) derart verbunden ist, dass es zum manuellen Bewegen des Kolbens (22) in der Pumpe (3) geeignet ist, so dass ein Unterdruck erzeugbar ist, so dass mit dem Unterdruck der Pumpe (3) durch die Verbindungsleitung (12) Gas aus dem Innenraum der zumindest einen Kartusche (4) evakuierbar ist,
wobei in der Kartusche (4) ein Zementpulver enthalten ist und das Vakuummischsystem einen von der Kartusche (4) separaten Behälter (2) aufweist, in dem eine Monomerflüssigkeit enthalten ist, wobei der Behälter (2) über eine Flüssigkeitsleitung (14, 16) mit der Kartusche (4) verbunden ist, wobei die Flüssigkeitsleitung (14, 16) an der Vorderseite der Kartusche (4) in die Kartusche (4) einmündet und die Verbindungsleitung (12) an der gegenüberliegenden Rückseite der Kartusche (4) in die Kartusche (4) einmündet, wobei der Kolben (22) in einem Hohlzylinder (20) axial beweglich gelagert ist,
**dadurch gekennzeichnet, dass**
die Kartusche (4), die Pumpe (3), der separate Behälter (2) und alle Leitungen (12, 14, 16) mit einem gemeinsamen Fußteil (5) fest und/oder lösbar verbindbar sind, wobei der Hohlzylinder (20) auf einer ersten Seite (24) durch einen Verschluss geschlossen ist oder bis auf eine Durchführung für eine mit dem Bedienelement (21) und dem Kolben (22) verbundene Stange (23) durch einen Verschluss (24) geschlossen ist und der Hohlzylinder (20) auf einer zweiten Seite offen ist, wobei ein Pumpraum (26) in dem Hohlzylinder (20) zwischen dem Kolben (22) und der ersten geschlossenen Seite (24) gebildet ist und wobei nach einem vollständigen Hub des Kolbens (22) innerhalb des Hohlzylinders (20) der Kolben (22) so verschoben ist, dass das Volumen des vom Hohlzylinder (20), dem Verschluss (24) und dem Kolben (22) gebildeten Pumpraums (26) mindestens gleich dem Volumen des Innenraums der zu evakuierenden Kartusche (4) ist.

2. Vakuummischsystem nach Anspruch 1, **dadurch gekennzeichnet, dass** die Pumpe (3) im Inneren einen gasdichten Pumpraum (26) aufweist und in der Pumpe (3) der bewegliche Kolben (22) angeordnet ist, der den Pumpraum (26) begrenzt, wobei der Kolben (22) manuell in einer Richtung, bevorzugt unidirektional, antreibbar ist, so dass durch die Bewegung des Kolbens (22) der Pumpraum (26) vergrößert wird und mit dem dadurch im Pumpraum (26) entstehenden Unterdruck der Innenraum der zumindest einen Kartusche (4) durch die Verbindungsleitung (12) evakuierbar ist.

3. Vakuummischsystem nach Anspruch 2, **dadurch gekennzeichnet, dass** die Volumenvergrößerung des Pumpraums (26) mindestens so groß ist wie das freie Volumen des Innenraums der Kartusche (4), bevorzugt die Volumenvergrößerung des Pumpraums (26) mindestens so groß ist wie die Summe des Volumens des Innenraums der Kartusche (4) und des Volumens der Verbindungsleitung (12) und des Volumens der Flüssigkeitsleitung (14, 16) zwischen dem Innenraum der Kartusche (4) und dem Behälter (2) und des Volumens einer mit dem Zementpulver in der Kartusche (4) zu mischenden Monomerflüssigkeit in dem Behälter (2).

4. Vakuummischsystem nach einem der vorangehenden Ansprüche, **dadurch gekennzeichnet, dass**
die Flüssigkeitsleitung (14, 16), die zwischen dem separaten Behälter (2) und dem Innenraum der Kartusche (4) vorgesehen ist, eine nach oben weisende Schlaufe (16) aufweist, wobei der höchste Punkt der Schlaufe (16) oberhalb einer Einmündung unter einem Monomer-Behälter (40) liegt, der in dem separaten Behälter (2) angeordnet ist.

5. Vakuummischsystem nach einem der vorangehenden Ansprüche, **dadurch gekennzeichnet, dass**
der Kolben (22) über eine Stange (23) mit dem Bedienelement (21) verbunden ist und vorzugsweise der Kolben (22) durch Drücken des Bedienelements (21) in der Pumpe (3) zu bewegen ist.

6. Vakuummischsystem nach einem der vorangehenden Ansprüche, **dadurch gekennzeichnet, dass**
in dem Innenraum der Kartusche (4) ein beweglicher Austragskolben (9, 10) zum Austragen des gemischten Knochenzements aus der Kartusche (4) angeordnet ist, wobei der Austragskolben (9, 10) vorzugsweise lösbar arretiert oder arretierbar ist, um eine Bewegung des Austragskolbens (9, 10) unter Einwirkung des Unterdrucks zu verhindern.

7. Vakuummischsystem nach Anspruch 6, **dadurch gekennzeichnet, dass**
der Austragskolben (9, 10) einen Durchgang mit einer gasdurchlässige Porenscheibe aufweist, die für das Zementpulver undurchlässig ist, wobei der Durchgang mit der Porenscheibe den Innenraum der Kartusche (4) mit der Umgebung gasdurchlässig verbindet, wobei der Durchgang gasdicht verschließbar ist, vorzugsweise mit einem Dichtungskolben (10) des Austragskolbens (9, 10) gasdicht verschließbar ist.

8. Vakuummischsystem nach einem der vorangehenden Ansprüche, **dadurch gekennzeichnet, dass**
die Kartusche (4) eine mit dem Zementpulver gefüllte Zementkartusche (4) ist, wobei der Behälter (2) durch ein zu öffnendes Trennelement flüssigkeitsundurchlässig mit dem Innenraum der Zementkartusche (4) verbunden ist und/oder der Innenraum der Zementkartusche (4) mit der Pumpe (3) gasdurchlässig verbunden ist oder verbindbar ist.

9. Vakuummischsystem nach einem der vorangehenden Ansprüche, **dadurch gekennzeichnet, dass**
die Pumpe (3), der separate Behälter (2) und alle Leitungen (12, 14, 16) fest mit dem Fußteil (5) verbunden sind und die Kartusche (4) lösbar mit dem Fußteil (5) verbunden ist.

10. Vakuummischsystem nach einem der vorangehenden Ansprüche, **dadurch gekennzeichnet, dass**
die Pumpe (3) aufgebaut ist mit
einem Hohlzylinder (20), wobei der Hohlzylinder (20) mit dem Innenraum der Kartusche (4) verbunden oder verbindbar ist,
einem gasdichten Verschluss (24) an einem Hohlzylinderende,
einem Kolben (22), der im Hohlzylinder (20) gasdicht, axial beweglich angeordnet ist,
mindestens einem manuell bedienbaren Bedienelement (21), mit dem der Kolben (22) in der Pumpe (3) manuell bewegbar ist,
wobei bei einem Bewegen des Kolbens (22) mit dem mindestens einem manuell bedienbaren Bedienelement (21) der Kolben (22) axial entgegengesetzt zum Verschluss (24) bewegbar ist und dadurch Gas aus dem Innenraum der Kartusche (4) evakuiert.

11. Verfahren zur Vermischung von Polymethylmethacrylat-Knochenzement in einem Innenraum einer Kartusche (4) eines Vakuummischsystems nach einem der vorangehenden Ansprüche, **dadurch gekennzeichnet, dass** eine Bewegung eines manuell angetriebenen Kolbens (22) einer Pumpe (3) des Vakuummischsystems zum Erzeugen eines Unterdrucks in der Pumpe (3) verwendet wird, wobei mit der derart angetriebenen Pumpe (3) der Innenraum der Kartusche (4) von oben evakuiert wird, wobei ein Zementpulver in dem Innenraum der Kartusche (4) enthalten ist und ein Gas aus dem Innenraum der Kartusche (4) mit der Pumpe (3) evakuiert wird, wobei mit dem Unterdruck eine Monomerflüssigkeit von unten in den Innenraum der Kartusche (4) eingeleitet wird und anschließend in dem Innenraum der Kartusche (4) die Monomerflüssigkeit mit dem Zementpulver in dem evakuierten Innenraum der Kartusche (4) zu einem Knochenzement gemischt wird.

12. Verfahren nach Anspruch 11, **dadurch gekennzeichnet, dass** das Volumen eines Pumpraums (26) der Pumpe (3) durch die manuelle Bewegung des Kolbens (22) vergrößert wird und durch den dadurch entstehenden Unterdruck der Innenraum der Kartusche (4) evakuiert wird.

13. Verfahren nach Anspruch 11 oder 12, **dadurch gekennzeichnet, dass** die Monomerflüssigkeit mit dem Unterdruck der Pumpe (3) in den Innenraum der Kartusche (4) gesaugt wird, wobei vorzugsweise die Monomerflüssigkeit von unten durch das Zementpulver gezogen wird.

14. Verfahren nach einem der Ansprüche 11 bis 13, **dadurch gekennzeichnet, dass** der Kolben (22) der Pumpe (3) manuell bewegt wird, wodurch ein Unterdruck gegenüber der umgebenden Atmosphäre in der Pumpe (3) erzeugt wird, dabei durch eine Verbindungsleitung (12) Gas von oben aus dem Innenraum der Kartusche (4) in den Hohlzylinder gesaugt wird und die Monomerflüssigkeit von unten in die Kartusche (4) und in das Zementpulver gesaugt wird, anschließend manuell oder motorgetrieben mit einer Mischvorrichtung (6) das Zementpulver mit der Monomerflüssigkeit vermischt wird,
anschließend die Kartusche (4) mit dem gemischten Zementteig entnommen wird und
durch axiale Bewegung eines Austragskolbens (9, 10) der Zementteig aus der Kartusche (4) gepresst wird.

15. Verfahren nach einem der Ansprüche 11 bis 14, **dadurch gekennzeichnet, dass** die Monomerflüssigkeit in einem von der Kartusche (4) separaten Behälter (2) angeordnet ist, wobei die Monomerflüssigkeit in einer Glasampulle (40) enthalten ist,
die Glasampulle (40) geöffnet wird, bevor der Kolben (22) manuell angetrieben wird, so dass eine flüssigkeitsdurchlässige Verbindung zwischen dem Innenraum der Kartusche (4) und dem Behälter (2) gebildet wird,
danach der Kolben (22) axial im Hohlzylinder (20) bewegt wird, wodurch ein Unterdruck gegenüber der umgebenden Atmosphäre erzeugt wird,
dabei durch die Verbindungsleitung (12) Gas aus dem Innenraum der Kartusche (4) in den Hohlzylinder (20) gesaugt wird und durch den in dem Innenraum der Kartusche (4) gebildeten Unterdruck Monomerflüssigkeit in die Kartusche (4) gesaugt wird.

## Claims

1. Vacuum mixing system for the mixing of polymethylmethacrylate bone cement, comprising
at least one cartridge (4) having an evacuable internal space for mixing of the bone cement,
a pump (3) with a plunger (22) that can be moved by hand to generate a low pressure, and
a connecting conduit (12) connecting the internal space of the at least one cartridge (4) to the pump (3) for generating a low pressure, whereby
whereby the pump (3) comprises an operating element (21) that can be operated from outside and is connected appropriately to the plunger (22) such that it is suitable for moving the plunger (22) in the pump (3) by hand such that a low pressure can be generated such that the low pressure of the pump (3) can be used to evacuate gas from the internal space of the at least one cartridge (4) through the connecting conduit (12),
whereby a cement powder is contained in the cartridge (4) and the vacuum mixing system comprising a container (2) separate from the cartridge (4), the container (2) containing a monomer liquid, whereby the container (2) is connected via a liquid line (14, 16) to the cartridge (4), whereby the liquid conduit (14, 16) opens at the front side of the cartridge (4) into the cartridge (4), and the connection conduit (12) opens on the opposite rear side of the cartridge (4) into the cartridge (4), wherein the plunger (22) is supported in a hollow cylinder (20) such as to be axially mobile, **characterized in that**
the cartridge (4), the pump (3), the separate container (2), and all connecting conduits (12, 14, 16) are connected to a common foot part (5) either affixed and/or in detachable manner, wherein the hollow cylinder (20) is closed on a first side or is closed by a closure (24) except for one feed-through for a rod (23) connected to the operating element (21) and the plunger (22), and the hollow cylinder (20) is open on a second side, whereby a pumping space (26) is formed in the hollow cylinder (20) between the plunger (22) and the first closed side (24) and wherein the plunger (22) is shifted appropriately after a full stroke of the plunger (22) in the hollow cylinder (20) such that the pumping space (26) formed by the hollow cylinder (20), the closure (24), and the plunger (22) is at least equal to the volume of the internal space of the cartridge (4) to be evacuated.

2. Vacuum mixing system according to claim 1, **characterised in that** the pump (3) comprises a gas-tight pumping space (26) on the inside and has the mobile plunger (22) limiting the pumping space (26) arranged inside the pump (3), whereby the plunger (22) can be driven by hand in a direction, preferably unidirectionally, such that the motion of the plunger (22) enlarges the pumping space (26) and the low pressure thus arising in the pumping space (26) allows the internal space of the at least one cartridge (4) to be evacuated through the connecting conduit (12).

3. Vacuum mixing system according to claim 2, **characterised in that** the volume enlargement of the pumping space (26) is at least equal to the free volume of the internal space of the cartridge (4), preferably the volume enlargement of the pumping space (26) is at least equal to the sum of the volume of the internal space of the cartridge (4) and the volume of the connecting conduit (12) and the volume of the liquid line (14, 16) between the internal space of the cartridge (4) and the container (2) and the volume of the monomer liquid in the container (2) to be mixed in the cartridge (4) with the cement powder.

4. Vacuum mixing system according to any one of the preceding claims, **characterised in that**
the liquid line (14, 16), which is provided between the separate container (2) an the internal space of the cartridge (4), that comprises an upward facing loop (16), whereby the apex of the loop (16) is situated above a junction under a monomer container (40) that is arranged in the separate container (2).

5. Vacuum mixing system according to any one of the preceding claims, **characterised in that**
the plunger (22) is connected to the operating element (21) by means of a rod (23) and, preferably, the plunger (22) is movable in the pump (3) by pushing the operating element (21).

6. Vacuum mixing system according to any one of the preceding claims, **characterised in that**
a mobile dispensing plunger (9, 10) for dispensing the mixed bone cement from the cartridge (4) is arranged in the internal space of the cartridge (4), whereby the dispensing plunger (9, 10) preferably is or can be locked in place in detachable manner in order to prevent the dispensing plunger (9, 10) from moving in response to the effect of the low pressure.

7. Vacuum mixing system according to claim 6, **characterised in that**
the dispensing plunger (9, 10) comprises a passage with a gas-permeable pore disk that is impermeable for the cement powder, whereby the passage with the pore disk connects the internal space of the cartridge (4) to the surroundings in gas-permeable manner, whereby the passage can be closed in gas-tight manner, preferably can be closed in gas-tight manner by means of a sealing plunger (10) of the dispensing plunger (9, 10).

8. Vacuum mixing system according to any one of the preceding claims, **characterised in that**
the cartridge (4) is a cement cartridge (4) filled with the cement powder, whereby the container (2) is connected, in liquid-impermeable manner, to the internal space of the cement cartridge (4) through a separating element that can be opened, and/or the internal space of the cement cartridge (4) is or can be connected to the pump (3) in gas-permeable manner.

9. Vacuum mixing system according to any one of the preceding claims, **characterised in that**
the pump (3), the separate container (2), and all connecting conduits (12, 14, 16) are affixed to the foot part (5) and the cartridge (4) is connected to the foot part (5) in detachable manner.

10. Vacuum mixing system according to any one of the preceding claims, **characterised in that**
the pump (3) is designed to comprise
a hollow cylinder (20), whereby the hollow cylinder (20) is or can be connected to the internal space of the cartridge (4);
a gas-tight closure (24) on one end of the hollow cylinder;
a plunger (22) that is arranged in the hollow cylinder (20) such as to be gas-tight and axially mobile;
at least one operating element (21) that can be operated by hand and can be used to move the plunger (22) in the pump (3) by hand;
whereby the plunger (22) can be moved axially in opposite direction with respect to the closure (24) when the plunger (22) is moved by the at least one operating element (21) that can be operated by hand and thus evacuates the gas from the internal space of the cartridge (4).

11. Method for the mixing of polymethylmethacrylate bone cement in an internal space of a cartridge (4) of a vacuum mixing system according to any one of the preceding claims, **characterised in that**
a motion of a manually driven plunger (22) of a pump (3) of the vacuum mixing system is used to generate a low pressure in the pump (3), whereby the pump (3) thus driven is used to evacuate the internal space of the cartridge (4) from above, whereby a cement powder is contained in the internal space of the cartridge (4) and a gas is evacuated from the internal space of the cartridge (4) by the pump (3), whereby a monomer liquid is introduced into the internal space of the cartridge (4) from below by the low pressure and then the monomer liquid and the cement powder is mixed in the evacuated internal space of the cartridge (4) to form a bone cement.

12. Method according to claim 11, **characterised in that**
the volume of a pumping space (26) of the pump (3) is enlarged by manually moving the plunger (22) and **in that** the internal space of the cartridge (4) is evacuated by the low pressure thus generated.

13. Method according to claim 11 or 12, **characterised in that** the monomer liquid is aspirated into the internal space of the cartridge (4) by the low pressure in the pump (3), whereby preferably the monomer liquid is pulled from below through the cement powder.

14. Method according to any one of the claims 11 to 13, **characterised in that** the plunger (22) of the pump (3) is moved by hand, whereby a low pressure with respect to the ambient atmosphere is generated in the pump (3);
whereby gas is aspirated from the internal space of the cartridge (4) from above through a connecting conduit (12) into the hollow cylinder and the monomer liquid is aspirated from below into the cartridge (4) and into the cement powder; followed by the cement powder being mixed, manually or motor-driven, with the monomer liquid by means of a mixing device (6);
followed by the cartridge (4) with the mixed cement dough being removed; and the cement dough being extruded from the cartridge (4) by moving a dispensing plunger axially (9, 10).

15. Method according to any one of the claims 11 to 14, **characterised in that** the monomer liquid is arranged in a container (2) that is separate from the cartridge (4), whereby the monomer liquid is contained in a glass ampoule (40); the glass ampoule (40) is opened before the plunger (22) is moved by hand such that a liquid-permeable connection is established between the internal space of the cartridge (4) and the container (2);
followed by the plunger (22) being moved axially in the hollow cylinder (20), whereby a low pressure with respect to the ambient atmosphere is generated; whereby gas is aspirated from the internal space of the cartridge (4) through the connecting conduit (12) into the hollow cylinder (20), and monomer liquid is aspirated into the cartridge (4) through the low pressure formed in the internal space of the cartridge (4).

## Revendications

1. Système de mélange sous vide pour le mélange d'un ciment osseux de polyméthyl méthacrylate présentant
au moins une cartouche (4) avec un espace intérieur pouvant être vidé pour le mélange du ciment osseux,
une pompe (3) avec un piston (22) pouvant être actionné manuellement pour la création d'une dépression et
une conduite de raccordement (12) qui relie l'espace intérieur de l'au moins une cartouche (4) avec la pompe (3) pour la création de la dépression,
où
la pompe (3) présente un élément d'actionnement (21) pouvant être actionné de l'extérieur qui est relié avec le piston (22) de telle manière qu'il est approprié pour le déplacement manuel du piston (22) dans la pompe (3), de sorte qu'une dépression peut être créée, de sorte qu'avec la dépression de la pompe (3), du gaz peut être évacué hors de l'espace intérieur de l'au moins une cartouche (4) par la conduite de raccordement (12),
où une poudre de ciment est contenue dans la cartouche (4) et le système de mélange sous vide présente un récipient (2) séparé de la cartouche (4) dans lequel est contenu un liquide de monomère, où le récipient (2) est relié ave la cartouche (4) par le biais d'une conduite de liquide (14, 16), où la conduite de liquide (14, 16) débouche sur le côté avant de la cartouche (4) dans la cartouche (4) et la conduite de raccordement (12) débouche sur le côté arrière se situant en face de la cartouche (4) dans la cartouche (4), où le piston (22) est logé mobile axialement dans un cylindre creux (20),
**caractérisé en ce que**
la cartouche (4), la pompe (3), le récipient (2) séparé et toutes les conduites (12, 14, 16) peuvent être reliés solidement, et/ou de manière amovible, avec une partie de socle (5) commune, où le cylindre creux (20) est fermé par un obturateur sur un premier côté (24) ou est fermé par un obturateur (24) jusqu'à un passage pour une tige (23) reliée avec l'élément d'actionnement (21) et le piston (22) et le cylindre creux (20) est ouvert sur un deuxième côté, où la chambre de la pompe (26) est formée dans le cylindre creux (20) entre le piston (22) et le premier côté (24) fermé et où, après un cycle complet du piston (22) à l'intérieur du cylindre creux (20), le piston (22) est décalé à tel point que le volume de la chambre de pompe (26), formé par le cylindre creux (20), l'obturateur (24) et le piston (22), est au moins égal au volume de l'espace intérieur de la cartouche (4) à vider.

2. Système de mélange sous vide selon la revendication 1, **caractérisé en ce que** la pompe (3) présente une chambre de pompe (26) étanche vis-à-vis des gaz à l'intérieur et le piston (22) mobile, qui délimite la chambre de pompe (26), est disposé dans la pompe (3), où le piston (22) peut être entraîné manuellement dans une direction, de préférence unidirectionnelle, de sorte que la chambre de pompe (26) est agrandie par le déplacement du piston (22) et, avec la dépression créée ainsi dans la chambre de pompe (26), l'espace intérieur de l'au moins une cartouche (4) peut être évacué par la conduite de raccordement (12).

3. Système de mélange sous vide selon la revendication 2, **caractérisé en ce que** l'agrandissement en volume de la chambre de pompe (26) est au moins aussi important que le volume libre de l'espace intérieur de la cartouche (4), de préférence, l'agrandissement en volume de la chambre de pompe (26) est au moins aussi important que la somme du volume de l'espace intérieur de la cartouche (4), et du volume de la conduite de raccordement (12), et du volume de la conduite de liquide (14, 16) entre l'espace intérieur de la cartouche (4) et le récipient (2) et du volume d'un liquide de monomère à mélanger dans le récipient (2) avec la poudre de ciment dans la cartouche (4).

4. Système de mélange sous vide selon l'une des revendications précédentes, **caractérisé en ce que**
la conduite de fluide (14, 16) qui est prévue entre le récipient (2) séparé et l'espace intérieur de la cartouche (4), présente une boucle (16) orientée vers le haut, où le point le plus élevé de la boucle (16) se situe au-dessus d'une embouchure sous un récipient de monomère (40), qui est disposé dans le récipient (2) séparé.

5. Système de mélange sous vide selon l'une des revendications précédentes, **caractérisé en ce que**
le piston (22) est relié avec l'élément d'actionnement (21) par le biais d'une tige (23) et le piston (22) peut être de préférence déplacé par la pression de l'élément d'actionnement (21) dans la pompe (3).

6. Système de mélange sous vide selon l'une des revendications précédentes, **caractérisé en ce**
**qu'**un piston d'évacuation (9, 10) mobile est disposé dans l'espace intérieur de la cartouche (4) pour l'évacuation du ciment osseux mélangé hors de la cartouche (4), où le piston d'évacuation (9, 10) peut être en butée de préférence de manière amovible ou peut être mis en butée afin d'empêcher un déplacement du piston d'évacuation (9, 10) sous l'effet de la dépression.

7. Système de mélange sous vide selon la revendication 6, **caractérisé en ce que** le piston d'évacuation (9, 10) présente un passage avec un disque poreux perméable aux gaz, qui ne laisse pas passer la poudre de ciment, où le passage avec le filtre poreux relie, en étant perméable aux gaz, l'espace intérieur de la cartouche (4) avec l'environnement, où le passage peut être fermé de manière étanche aux gaz, de préférence, de manière étanche aux gaz avec un piston d'étanchéité (10) du piston d'évacuation (9, 10).

8. Système de mélange sous vide selon l'une des revendications précédentes, **caractérisé en ce que**
la cartouche (4) est une cartouche de ciment (4) remplie avec la poudre de ciment, où le récipient (2) est relié en ne laissant pas passer de liquide avec l'espace intérieur de la cartouche de ciment (4) par un élément de séparation à ouvrir et/ou l'espace intérieur de la cartouche de ciment (4) est relié ou peut être relié en étant perméable aux gaz avec la pompe (3).

9. Système de mélange sous vide selon l'une des revendications précédentes, **caractérisé en ce que**
la pompe (3), le récipient (2) séparé et toutes les conduites (12, 14, 16) sont reliés solidement avec la partie de socle (5) et la cartouche (4) est reliée avec la partie de socle (5) de manière amovible.

10. Système de mélange sous vide selon l'une des revendications précédentes, **caractérisé en ce que**
la pompe (3) est construite avec
un cylindre creux (20), où le cylindre creux (20) est relié ou peut être relié avec l'espace intérieur de la cartouche (4),
un obturateur (24) étanche aux gaz sur une extrémité du cylindre creux,
un piston (22) qui est disposé de manière étanche aux gaz en étant mobile axialement dans le cylindre creux (20),
au moins un élément d'actionnement (21) pouvant être actionné manuellement avec lequel le piston (22) peut être déplacé manuellement dans la pompe (3),
où, lors d'un déplacement du piston (22) avec l'au moins un élément d'actionnement (21) pouvant être actionné manuellement, le piston (22) peut être déplacé axialement dans le sens contraire par rapport à l'obturateur (24) et évacue ainsi du gaz hors de l'espace intérieur de la cartouche (4).

11. Procédé de mélange de ciment osseux de polyméthyl méthacrylate dans un espace intérieur d'une cartouche (4) d'un système de mélange sous vide selon l'une des revendications précédentes, **caractérisé en ce**
**qu'**un déplacement d'un piston (22) entraîné manuellement d'une pompe (3) du système de mélange sous vide est employé pour la création d'une dépression dans la pompe (3), où l'espace intérieur de la cartouche (4) est évacué par le haut avec la pompe (3) ainsi entraînée, où une poudre de ciment est contenue dans l'espace intérieur de la cartouche (4) et un gaz est évacué hors de l'espace intérieur de la cartouche (4) avec la pompe (3), où un liquide de monomère est introduit à partir du bas dans l'espace intérieur de la cartouche (4) avec la dépression et ensuite le liquide monomère est mélangé dans l'espace intérieur de la cartouche (4) avec la poudre de ciment dans l'espace intérieur évacué de la cartouche (4) pour donner un ciment osseux.

12. Procédé selon la revendication 11, **caractérisé en ce que**
le volume d'une chambre de pompe (26) de la pompe (3) est augmenté par le déplacement manuel du piston (22) et l'espace intérieur de la cartouche (4) est évacué par la dépression ainsi générée.

13. Procédé selon la revendication 11 ou la revendication 12, **caractérisé en ce que**
le liquide de monomère est aspiré dans l'espace intérieur de la cartouche (4) avec la dépression de la pompe (3), où, le liquide de monomère est de préférence absorbé par le bas par la poudre de ciment.

14. Procédé selon l'une des revendications 11 à 13, **caractérisé en ce que**
le piston (22) de la pompe (3) est déplacé manuellement, ce par quoi une dépression vis-à-vis de l'atmosphère environnante est créée dans la pompe (3),
ce faisant, du gaz est aspiré par le haut hors de l'espace intérieur de la cartouche (4) par une conduite de raccordement (12) dans le cylindre creux et le liquide de monomère est absorbé par le bas dans la cartouche (4) et dans la poudre de ciment,
ensuite, la poudre de ciment est mélangée avec le liquide de monomère manuellement ou avec un entraînement moteur,
ensuite, la cartouche (4) avec la pâte de ciment mélangée est prélevée et
la pâte de ciment est pressée hors de la cartouche (4) par un déplacement axial d'un piston d'évacuation (9, 10).

15. Procédé selon l'une des revendications 11 à 14, **caractérisé en ce que** le liquide de monomère est disposé dans un récipient (2) séparé de la cartouche (4), où le liquide de monomère est contenu dans une ampoule en verre (40),
l'ampoule en verre (40) est ouverte avant que le piston (22) ne soit entraîné manuellement de sorte qu'une liaison laissant passer le liquide est établie entre l'espace intérieur de la cartouche (4) et le récipient (2),
ensuite, le piston(22) est déplacé axialement dans le cylindre creux (20), ce par quoi une dépression vis-à-vis de l'atmosphère environnante est créée,
ce faisant, du gaz est aspiré hors de l'espace intérieur de la cartouche (4) dans le cylindre creux (20), et du liquide de monomère est aspiré dans la cartouche (4) par la dépression créée dans l'espace intérieur de la cartouche (4).
